# EUROPEAN PATENT SPECIFICATION

(11) **EP 3 310 326 B1**
(45) Date of publication and mention of the grant of the patent: **04.08.2021**
(21) Application number: 16736320.9
(22) Date of filing: 22.06.2016
(51) Int. Cl.: A61K 8/27, A61Q 19/00, A61Q 19/08, A61K 8/02, A61K 8/81, A61K 8/34, A61Q 19/02, A61K 8/37, A61K 9/00, A61K 47/32, A61K 9/70, A61K 31/05, A61K 31/192, A61K 31/455, A61K 31/60

(54) **MULTI-LAYER TOPICALLY-APPLIED ARTICLE FOR PROVIDING A BENEFIT TO SKIN**
MEHRSCHICHTIGER, TOPISCH ANGEWANDTER ARTIKEL ZUR BEREITSTELLUNG EINES NUTZENS FÜR DIE HAUT
ARTICLE MULTICOUCHE APPLIQUÉ PAR VOIE TOPIQUE POUR FOURNIR UN BIENFAIT SUR LA PEAU

(30) Priority: 22.06.2015 US 201562182888 P
(43) Date of publication of application: 25.04.2018
(73) Proprietor: Johnson & Johnson Consumer Inc., Skillman, NJ 08558 (US)
(72) Inventor: BINNER, Curt, Skillman, NJ 08558 (US); PATURI, Jyotsna, Skillman, NJ 08558 (US); TRAN, Bao, T., Skillman, NJ 08558 (US); WU, Jeffrey, M., Skillman, NJ 08558 (US)
(74) Representative: Carpmaels & Ransford LLP
(86) International application number: PCT/US2016/038642
(87) International publication number: WO 2016/209880

(56) References cited:
- EP-A1- 2 090 293
- WO-A2-2006/009987
- WO-A2-2012/087517
- US-A1- 2008 124 381

## Description

### FIELD OF THE INVENTION

The present invention relates to a self-adhesive, multi-layer article that is applied to an application site on the skin of a consumer, which provides a cosmetic and/or therapeutic benefit to the application site and which is readily removable with water.

### BACKGROUND OF THE INVENTION

WO 2012/087517 concerns personal care products, including cosmetics, are provided as solid, water-soluble polymeric films which adhere to a human integument when wetted, and such films having plural laminated layers, at least two of which possess different cosmetic or therapeutic characteristics.

EP2090293 concerns water-dispersible or water-soluble polymer films incorporating at least one cosmetic or dermatologic active agent and a method for the production thereof.

WO 2006/009987 concerns a system, a kit and methods for delivering an effective amount of a labile active to the skin, comprising a composition comprising an effective amount of a labile active agent incorporated into a water-soluble polymeric film and an additive composition capable of dissolving the water-soluble polymeric film. US 2008/124381 concerns integratable adhesive films containing a mixture of high molecular weight and low molecular weight water soluble components; and a pharmaceutically or cosmetically active ingredient.

A variety of personal or consumer healthcare ingredients or compositions may be applied to the skin to affect the health or condition or appearance or any other aspect of the skin. They are often applied to the skin in the form of skin care preparations. Skin care preparations include emulsions (hydrous creams and lotions), pastes and jellies, anhydrous creams and lotions, ointments, hydro/alcoholic solutions or gels, sprays, serums, and sticks. These preparations are left on the skin for a period of time to allow the various ingredients, compositions, or actives therein to diffuse into the skin. An issue with these preparations is the difficulty with leaving them on the skin for long periods of time, such as all day or overnight. If the site of the application contacts other surfaces or articles, such as clothing, or, during overnight use, bedding (such as sheets or pillows), the preparation may rub off and stain the surface or article it contacts. In addition, lotions, creams, or ointments are often tacky, viscous materials which could pick up dust or dirt or other debris when left exposed on the skin of the user, causing negative aesthetic effects for the user, and potential discomfort as well. Finally, such preparations must be removed from the skin, and removal often may require more than simply washing the face in the ordinary manner, and may require use of more than simply water, such as cleansers and / or other compositions, washcloths, tissues, cotton balls, etc.

Skin care products may be delivered in combination with a patch. For instance, the topical ingredient or composition may be dissolved in the adhesive that holds the patch to the treatment site and delivered to the treatment site over time. Such patches typically are formed of nonwoven, mesh, etc., materials that are general not dissolvable. Therefore, an issue with these forms of application of topical ingredients or compositions to the skin of a user is removal of the sheeting or patch at the end of the treatment time. In particular, a user often must forcibly tear the patch away from the skin to break the adhesive bond of the patch to the skin. This tearing often causes discomfort, and even pain, for the user. The pain may be due to sensitive or fragile skin, where any pulling of the sticky patch can induce skin injury. Also, in sections of the skin with significant amounts of hair, forceful removal of an adherent topical patch may also cause undesired removal of hair, thereby also causing pain to the user.

Dissolvable films formulations are widely available in patent literature as solutions to the above-noted disadvantages of patches that must be peeled away from the user's skin. Typical formulations or patents describe a substantially dry film comprised of one or more polymers as mechanical support (film formers), and one or more adhesive or tacking substances to permit adhesiveness of the dissolvable film upon application of the dry film onto moist skin or upon application of the dry film to skin in conjunction with an appropriate solvent.

Single-layer, rapidly dissolving films for delivering an active agent are known. The films include a film forming binder, a rapidly dissolving polymeric material, and an active agent contained within the film layer. The films are non-tacky during storage and prior to application and thus will not stick to skin or mucosal membranes upon placement thereon.

In summary, skin is complex and providing a benefit to the skin, including the treatment of skin, including the delivery of actives or other topical ingredients or compositions to the skin using viscous preparations alone or in combination with patches has limitations in effectiveness or aesthetics, or comfort for the user. There remains a need for topically applied articles and / or delivery systems that are easy to use (including easy to apply), may be left on the user for long periods, and yet be readily removable once delivery has been completed or when desired.

### SUMMARY OF THE INVENTION

According to the present invention, an article suitable for providing a cosmetic and/or therapeutic benefit to the skin upon topical application of the article thereto is provided. The article comprises a first top layer having a top surface facing outwardly from the skin and a second bottom surface opposite the top surface facing towards the skin. The article also comprises a second bottom skin-contacting layer comprising a first surface facing and adhered to the bottom surface of the first top layer and a second outwardly-facing surface for contacting and adhering the article to the skin when the article is applied thereto. The bottom skin-contacting layer comprises at least one benefiting agent selected from the group consisting of a cosmetic agent and a therapeutic agent wherein the at least one benefiting agent comprises hexylresorcinol. Each of the first top layer and second bottom layer comprises a water-soluble film former and the article is readily removable from the skin upon application of water thereto. The second bottom layer further comprises an effective amount of a glyceride emulsifier to enhance transport of the benefiting agent therefrom. The invention also provides a non-therapeutic method of providing a cosmetic benefit to skin, the method comprising:
applying an article as defined in the claims, wherein the at least one benefiting agent comprises hexylresorcinol, to an application site on the skin;
maintaining the article in place for at least half an hour; and
removing the article from the application site by application of water to the article.

These and other features and advantages of the present invention will be readily apparent from the following detailed description of the invention, the scope of the invention being set out in the appended claims.

### BRIEF DESCRIPTION OF THE DRAWINGS

The detailed description will be better understood in conjunction with the accompanying drawings, wherein like reference characters represent like elements, as follows:
FIGURE 1 is a plan view of an article of the present invention; and
FIGURE 2 is a cross-sectional view of the article of FIGURE 1 along line 2-2.
FIGURE 3 is a graphical presentation of the results of Example 16.

### DETAILED DESCRIPTION OF THE INVENTION

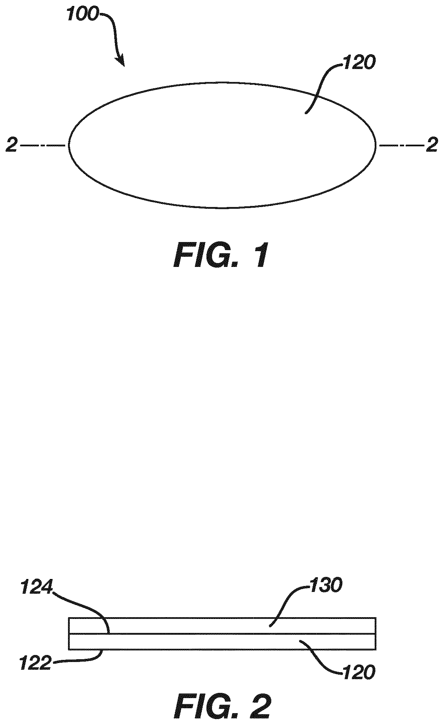

Article 100 of the present invention is shown in FIGURES 1 and 2. In the embodiment illustrated in FIGURES 1 and 2, article 100 has bottom, skin-contacting layer 120, and top layer 130, having top surface 132 facing outwardly from the skin and bottom surface 134 facing towards the skin. Skin-contacting layer 120 has first, skin-contacting surface 122 that adheres to the skin of the user when applied thereto. Skin-contacting layer 120 has second surface 124 opposite first, skin-contacting surface 122, fixedly attached to bottom surface 134 of top layer 130.

The topically-applied article of the present invention, as shown above and further described herein below, preferably is easy to apply, comfortable to wear, and readily removable, i.e., disintegrable and/or dissolvable, upon application of water thereto. It will be appreciated that reference to "topical" herein shall be understood to include skin, mucosa, and other application sites to which an article of the present invention may be applied.

In accordance with a more particular aspect of the present invention, the outwardly-facing top surface of the top layer, i.e., the surface facing away from the skin of the consumer to whom the article is adhered, is not tacky so that the topically-applied article stays in place at the application site, does not stick to another article and does not gather dust, dirt or other debris from the immediately surrounding area. Preferably, the bottom layer of the topically-applied article is self-adhesive to skin, i.e, adheres to skin, or at least readily adheres to skin with a nominal level of hydration already provided with the article.

A topically-applied article 100, illustrated in FIGURES 1 and 2, is provided in a form that is comfortable, easy to apply to the application site, remains in place for an extended period of time, e.g., at least half an hour, or at least one hour, or at least about six (6) to eight (8) hours, or at least about twelve (12) hours, or about twenty four (24) hours, if desired. The topically-applied article is readily removable upon application of water thereto. By readily removable, it is meant that the article may dissolve or disintegrate upon application of water to the article, such that it may be removed from the skin without scrubbing, or the like. Article 100 preferably is a topically-applied skin care article, patch, applique, etc. (hereinafter "article" for the sake of convenience, without intent to limit) that preferably is relatively flexible.

An article of the present invention preferably is relatively thin and flexible, as described in further detail below, so that it preferably readily conforms to the user's skin and is comfortable to wear, both because of the flexibility and conformability, as well as from the thinness. An article of the present invention intended for extended wear preferably is also formed to be aesthetically elegant without peeling, wrinkling, cracking, or appearing greasy or tacky, or otherwise unpleasant or unsightly in nature. The article preferably is formed with sufficient rigidity and integrity to be able to withstand normal use when on the skin. For instance, an article of the invention preferably is formed with sufficient strength to stay intact on the skin when exposed to normal external forces that the skin may experience, e.g., rubbing of clothing, pillow, etc.

Preferably, an article of the present invention is relatively easy to apply to the desired application site. If desired, an article of the present invention may be formed to have structural integrity. As used herein, structural integrity is to be understood as the physical capability of the article to maintain a substantially monolithic form or structure and to resist tearing or fracture while being manipulated independent of a substrate, and preferably while being applied to an application site. For instance, an article of the present invention may be sufficiently robust to permit handling for a desired purpose separate from any supporting substrate, i.e., preferably the article is self-supporting. If a supporting substrate is used, the article or article preferably is removable from the substrate for use independent of the substrate.

It will be appreciated that structural integrity of an article of the present invention preferably also contributes to the article's ability to remain intact during manipulation and use, and to conform to the contours of the application site to which it is applied, as discussed in further detail below. For instance, it is desirable that the article have sufficient structural integrity so that the article does not readily tear when removed from a substrate, manipulated, worn, or otherwise used. It will be appreciated that selection of one or more film formers that contribute to a product's ability to achieve a pliable, cohesive, and continuous covering on an application site such as skin, is one manner of achieving the desired structural integrity of an article of the present invention. Additionally, or alternatively, selection of one or more plasticizers for producing or promoting plasticity and flexibility and reducing brittleness, is another manner of achieving the desired structural integrity of an article of the present invention.

The structural integrity of an article of the present invention typically may be correlated with the tensile strength and thickness of the article. In connection with the present invention, structural integrity typically increases as thickness and yield strength increase. However, such properties must be balanced with their effect on whether the article is comfortable to be worn, as discussed in further detail below. Tensile strength contributes to the structural integrity of an article formed in accordance with principles of the present invention for such purposes as handling and/or removing the article from a substrate. Tensile strength affects, *inter alia,* whether the article resists being fractured when being handled and/or removed from a substrate. For instance, an article of the present invention preferably has an elastic modulus of about 1,500psi to about 5,000psi. An elastic modulus of about 2,500psi has been found in one embodiment to provide the desired stiffness to be comfortable during use. Typical samples with a ¾ inch (1.905 cm) width and a 0.1mm thickness have a rupture-strength of about 2 lbf (pound force), although it will be appreciated that a useful range of rupture strengths is from about 0.51bf to about 51bf. The thickness of the article also affects structural integrity. For instance, the thickness of an article of the present invention may be between about 0.05mm to about 2mm, and preferably between about 0.05mm and 0.3mm. A thickness of approximately 0.1mm has been found to provide the desired mechanical properties for handling, applying, and ultimately removing the article, such that the article maintains its structural integrity throughout such use, as well as while being worn on a given application site, as discussed in further detail below.

In accordance with one aspect of the present invention, an article of the present invention is self-adhesive, i.e., it adheres to a user's skin upon contact with the skin, preferably without additional steps, such as addition of another composition, such as water. The unique adhesive properties may be imparted by at least one water-soluble carbohydrate, such as saccharides (monosaccharides or disaccharides or oligosaccharides or polysaccharides or mixtures thereof). Saccharides have the chemical formula Cx(H₂O)y with the chemical structure H(CHOH)nC=O(CHOH)mH. Examples include starch derived from different plant sources, high amylase and high amylopectin varieties. The term "starch," as used herein, also includes water-soluble film-forming polymer materials derived from starch, including starch derivatives such as starch hydrolate products, modified starches, modified starch derivatives, and maltodextrins. Water soluble carbohydrate oligomers are preferred. Suitable water soluble carbohydrate oligomers include xylose, ribose, glucose, mannose, galactose, fructose, dextrose, polydextrose, sucrose, maltose, corn syrup solids, palatin, sorbitol, xylitol, mannitol, maltitol, lactitol, xanthan, maltodextrin, galactomanan, tragacanth, manitol, lactitol, oligisaccharides and hydrocolloids, and mixtures thereof, such as corn syrup, honey, high fructose corn syrup, etc.

In an embodiment of the present invention in which the article is formed with corn syrup containing a mixture of water soluble carbohydrates of glucose and its polymers, the adhesive properties of the film are generally tied to the moisture content of the substance in which the water soluble carbohydrates is contained. In such embodiment, the article preferably has a moisture content in the range of about 6% to about 15% by weight to provide adequate adhesion. Below such range typically does not provide sufficient adhesion, and above such range typically causes the film to lose too much structure.

The advantages of water-soluble carbohydrates are their in-situ retention of moisture or water through the interactions of both intermolecular and hydrogen bonding. The presence of an appropriate amount of water, e.g., about 6%-15% by weight, surprisingly provides the article an adhesion sufficient for the article to stick to the application site without falling off. The water-soluble carbohydrates also impart a soft pliable texture to allow the article to conform to any surface contour or shape, while maintaining the proper adhesion.

In one embodiment, polysaccharides can be used. Suitable polysaccharides are polysaccharides of the non-sweet, colloidally-soluble types, such as natural gums, for example, gum arabic, starch derivatives, dextrinized and hydrolyzed starches, and the like. A suitable polysaccharide is a water dispersible, modified starch.

In one embodiment, water soluble bioadhesive polymers can be used for enhancing skin adhesive property. Examples useful for the invention include, but are not limited to, cellulose and its derivatives, polyvinyl pyrrolidone, water soluble celluloses, polyvinyl alcohol, ethylene maleic anhydride copolymer, methylvinyl ether maleic anhydride copolymer, acrylic acid copolymers, anionic polymers of methacrylic acid and methacrylate, cationic polymers with dimethyl-aminoethyl ammonium functional groups, polyethylene oxides, water soluble polyamide or polyester, polyethylene glycol, water soluble acrylic polymers, water soluble polyesters, hydroxyalkyl starches, casein, gelatin, solublized proteins, polyacylamide, polyamines, polyquaterniuma mines, styrene maleic anhydride resins, polyethyelene amines, The water soluble carbohydrate can form hydrogen or covalent bonding to the water soluble or hydrophilic polymer in the film.

In accordance with one aspect of the present invention, the adhesive quality of an article of the present invention is preferably capable of fixing the article to the skin of a user for an extended period of time, as discussed herein above, without irritating the skin. Preferably, the article is capable of adhering to the application site for as long as reasonable and/or indicated to have an article in place at such site. Thus, an upper temporal limit to adhesion time is not important, since the user or wearer typically will want to remove the article before the article would naturally wear off of the application site on its own. Typically, the amount of time an article of the present invention is to adhere to a given application site is dictated by the amount of time the application area can withstand not being exposed to water. For instance, it will be appreciated that some surgical sites are not to be exposed to water for extended periods of time, such as several days. Articles for application to such sites should accordingly be capable of adhering to such site for so long as the site is not exposed to water, if desired. As may be appreciated, the adhesive preferably is selected for application onto a skin surface which typically is not considered to be moist, in contrast with mucosal tissue. It will be appreciated that by being capable of adhering to the user's skin, the article simply is capable of adhering, but need not necessarily adhere if such property is not desired or unnecessary for a particular application.

Because an article of the present invention preferably is formed to remain adhered to the application site for an extended period of time, as described above, the outwardly-facing top surface of the top layer preferably has desirable properties and features to facilitate such an intended use of the article. For instance, because an adhesive article or surface is designed to adhere to an application site, if the article is designed to adhere to an application site for an extended period of time, then an adhesive outwardly-facing surface may unintentionally or inadvertently adhere to another surface or object during use of the article. Such unintentional or inadvertent occurrence may cause the article to become dislodged, or, worse, disengaged (partially or even fully) from the application site. Moreover, it will be appreciated that an adhesive material typically attracts dust or dirt or other debris, which would likely be considered by the wearer to be unsightly and undesirable. Accordingly, it is preferable that the outwardly-facing top surface of the top layer of the article of the present invention is not adhesive. Thus, an article of the present invention, to remain adhered to an application site for an extended period of time, preferably has an outwardly-facing top surface of the top layer that is covered or otherwise rendered non-adhesive.

In one embodiment, a third non-adhesive, release layer is provided over the outwardly facing surface of the adhesive, skin contacting layer of an article of the present invention. It will be appreciated that if the non-adhesive release layer separates from the adhesive, skin-contacting layer (hereinafter, simply "skin-contacting layer" for the sake of simplicity) prior to application of the article to the skin, such separation would likely affect performance (e.g., appearance, tactile properties (the article would be sticky) of the article, and semi-occlusive performance (such as discussed below)). Thus, such non-adhesive outwardly-facing release layer preferably is formed to remain coupled with the adhesive skin-contacting layer until such time as the article is ready to be applied to the skin. The non-adhesive outwardly-facing release layer may be composed of a material that does not adhere to other objects upon contact therewith. Preferably, the adhesiveness of the outwardly facing layer does not change with water content or upon exposure to different environmental conditions.

If the outwardly-facing layer rubs against or is rubbed by something or otherwise contacts or is contacted by another surface or article, the article should not adhere to such surface or article. Likewise, the outwardly-facing release layer should be sufficiently coupled to the adhesive skin-contacting layer, as described above, that the outwardly-facing release layer does not become detached from the adhesive skin-contacting layer if the outwardly-facing release layer rubs against or is rubbed by something or otherwise contacts or is contacted by another surface or article.

The article may be tinted or pigmented to match the skin tone of the user so that it is aesthetically pleasing, or at least not unaesthetic or unsightly, when worn. The article may be formed to be clear to be discrete in situ. Further properties may be selected to render an article of the present invention visually discrete when in situ so that if the article is worn during the day its noticeability is minimized as much as possible. For instance, the thinner the article is, the less visible the article typically is. In addition, or alternatively, the color, texture (e.g., rough, slick, smooth, or otherwise textured such as an "orange peel" surface to match substantially the texture of the skin to which the article is applied so that the article is not starkly smooth relative to the skin with its natural imperfections), shine (shiny or dull depending on application site), etc., may be modified as desired to facilitate blending in of the article with the application site. Because an article of the present invention may be configured to be worn for an extended period of time (e.g., more than an hour, such as described above, and/or even overnight), the article preferably is formed or configured to be comfortable when worn. A variety of factors (individually or in any combination) may be considered in achieving the desired comfort and level of comfort, including, without limitation, tactile properties, material thickness (affecting not only durability, but also weight on the application site), and stiffness. Tactile properties that may contribute to comfort include smoothness, and / or stickiness of the adhesive used to adhere the article to a selected application site, etc. Additional tactile properties that may contribute to comfort include softness, smoothness, and texture of the film, such as determined by modulus of elasticity and coefficient of friction (rather than merely the aesthetic aspects of such properties).

Thickness affects a variety of additional factors, including stiffness - a stiffer article typically being less comfortable than a less stiff article. Material properties (a function of the composition of the material, independent of form) as well as structural properties (the form of the article) may affect the achievable comfort level of an article formed in accordance with principles of the present invention when worn by a user. It will be appreciated that all the desired properties for an article formed in accordance with principles of the present invention must be balanced, wherein some properties complementary, yet others have opposing dimensions. With regard to comfort, it will be appreciated that properties contributing to comfort must be balanced with properties contributing to structural integrity. There are at least three structural properties that affect "comfort": flexibility (about a single, bending direction; generally, flexibility is considered a combination of thickness and flexural modulus), stretchability (in a single axial direction; generally, stretchability is considered a combination of thickness and elastic modulus), and conformability (generally considered a combination of flexibility and physical shape in multiple directions, about complex surface). Comfort may be achieved by minimizing both the thickness and the elastic modulus. It will be appreciated that flexibility and stretchability are both functions of the elastic modulus of the material. More particularly, flexibility generally is dictated by the thickness of the material as well as the flexural modulus. Stretchability is a function of thickness and elastic modulus. When the material is thicker, stiffness increases (which property correlates with comfort) and flexibility and stretchability are reduced, generally adversely impacting comfort. The elastic modulus generally affects how rubbery or brittle a material is, and is tied to comfort because it determines flexibility of the material. Increasing the flexural or elastic modulus of a material makes the material less flexible or stretchable, respectively. Specifically, a higher flexural or elastic modulus results in a stiffer material, so the material consequently is less flexible and less stretchable. Given a constant flexural or elastic modulus, a higher material thickness will make the material less flexible or stretchable. As may be appreciated, comfort may be achieved by minimizing thickness of a given film to the lowest practical limit. The lower limit is dictated by providing enough structure to handle and manipulate the article and to facilitate application and removal of the article. From a material standpoint, the elastic modulus is most strongly linked to comfort. The lower the elastic modulus, the more comfortable the article typically is. An elastic modulus of from about 500PSI to about 10,000 PSI provides an acceptable degree of comfort for a user, with a more preferred range of elastic modulus of from about 1000 PSI to about 5000 PSI, with a preferred elastic modulus of about 2500 PSI. Conformability, such as the ability to conform to a given site (typically a surface with a complex curvature), not only involves flexibility, in general, but also relates to multidirectional flexibility and stretchability (e.g., so the article may stretch if placed over a joint). Conformability generally must be defined in terms of the physical shape or contour of the application site, and is determined with respect to a surface in conjunction with flexibility. An article may need to have a particular planar shape to be able to conform to a complex surface. Preferably, an article formed in accordance with principles of the present invention has substantially the same properties in all directions.

If an article formed in accordance with principles of the present invention is to remain on the application site for an extended period of time, such article preferably has a desired degree of breathability. Breathability may also be important for obtaining desired skin moisturization or proper skin moisture content balance for the functionality of the article in providing such benefit. Breathability relates to and is a function of oxygen exchange, which affects skin barrier as well as consumer perception. Breathability also is a function of water transmission. An article formed in accordance with principles of the present invention preferably is sufficiently breathable so that the skin moisture content remains balanced. Of course, if one of the desired outcomes of use of the present invention is to improve or to increase skin moisture content, then the breathability of the article preferably may be selected to facilitate such moisturization, as discussed in further detail below. A semi-occlusive film will at least partially inhibit water loss and therefore hold moisture within the skin. An article formed in accordance with principles of the present invention preferably provides resistance to moisture transmission, and may have a moisture transmission rate of approximately 50-150 grams of water per hour per square meter. Such article has been found to block or occlude evaporation that would occur without a film barrier by approximately 87%.

An article formed in accordance with principles of the invention, as further described below, may be semi-occlusive (preferably approximately 85% occlusive) not only to maintain breathability, but also to provide other benefits discovered to result from covering the application site with a semi-occlusive article. If an article is formed with an outwardly facing release layer, then such release layer may further contribute to the overall semi-occlusive nature of the article. In particular, an outwardly-facing layer may function in conjunction with a hygroscopic skin-contacting layer. Once such a skin-contacting layer hydrates further, it may further lose structural integrity, and transform from a film-type substance to a gel phase without structural integrity independent of the outwardly-facing layer. The outwardly-facing layer thus essentially caps and contains the skin-contacting surface at the application site so that the skin-contacting surface can hydrate the application site.

An article of the present invention dissolves or disintegrates with only the addition of water. Preferably, no mechanical agitation is required to facilitate the removal of the article. Preferably, an article of the present invention preferably completely dissolves within the parameters of a typical consumer washing regimen for the application site if no article is present, so no additional washing time is required by the consumer. Preferably, an article of the present invention is quick-dissolving for ready removal from the application site on the user (when washing one's face, preferably less than about 5 minutes, and even less than about 1 minute, and even about 30 seconds after addition of a water thereto. It will be appreciated that a longer dissolution rate is acceptable for sites on other parts of the body that are typically washed for more than 5 minutes, but preferably not so long a dissolution time that scrubbing is required to achieve removal). With simulated cleansing water flow of about 1.2 m/s (4 ft / s) (parallel flow to surface of film), complete dissolution was measured in about 67 seconds with initial breach of the outer film surface occurring at about 30 seconds. In another embodiment, the film can be removed with a wet cloth, sheet, or pad made of woven or nonwoven materials.

As used herein the specification and the claims, the term "water-removable" and variants thereof means that the article is removed from a surface to which it is adhered in less than 90 seconds with a water flow rate of 1.2 m/s (4 feet/second (parallel to the top surface of the article) at room temperature (21° C).

The primary mechanical strength of the film is created by the film former (preferably polyvinyl alcohol (PVA) in the outwardly-facing layer), which typically is also selected based on its ability to permit ready breakdown of the article as desired. It will be appreciated that in one embodiment, the film former preferably is selected to achieve the desired ability to dissolve or disintegrate the article for removal upon application of water thereto, and may be the first component of the article composition that is selected, other components being selected to interact as desired with the already-selected film former. Flexibility is achieved by the addition of a plasticizer, such as glycerin, to the film former. Film formers and/or plasticizers typically are the primary contributors to structural integrity, and are typically a component of the composition used to form the outwardly-facing top layer with properties such as described above. Exemplary film formers and plasticizers are set forth in greater detail below. Looking at solids content, PVA can be 70% by weight of the dry ingredients of an article formed in accordance with principles of the present invention, with glycerin at 20% by weight. The glycerin can range from as little as 10% to 30% by weight of the article, and the PVA can be as much as 90% by weight of the dry ingredients.

It will be appreciated that skin-contacting layer 120 and outwardly-facing layer 130 are selected so that they are coupled together to form an integral structure. Preferably, skin-contacting layer 120 and outwardly-facing layer 130 not only do not detach from each other, but also do not shift with respect to each other during manipulation of article 100. For instance, if something rubs against the outwardly-facing layer 130 or outwardly-facing layer 130 rubs against something, outwardly-facing layer 130 should be coupled strongly enough to skin-contacting layer 120 so that outwardly-facing layer 130 does not detach from skin-contacting layer 120. In one embodiment, the strength of adhesion of article 100 to the application site is selected to be weaker than the strength of adhesion of skin-contacting layer 120 and outwardly-facing layer 130 to each other. The bond between the two layers essentially creates a single product that is inseparable and functions as a single, unitary, monolithic article.

In accordance with one aspect of the present invention, skin-contacting layer 120 and outwardly-facing layer 130 are formed using the same solvent, so that whether a two-layer article 100 is formed when compositions forming skin-contacting layer 120 and outwardly-facing layer 130 are both in a wet state, or when one of the compositions of skin contacting layer 120 and outwardly-facing layer 130 is in a wet state and the other of the compositions of skin-contacting layer 120 and outwardly-facing layer 130 is in a dry state, there still will be some amount of mixing on a molecular level between the two components at the interface of the layers. Accordingly, the raw materials of skin-contacting layer 120 and outwardly-facing layer 130 are preferably selected so that they intermix to an extent that skin-contacting layer 120 and outwardly-facing layer 130 remain coupled together and do not separate before or during use. Preferably, both layers are water soluble so that some level of mixing (preferably on the molecular level) may occur to result in the desired bonding between the layers. However, it will be appreciated that intermixing of the materials on a molecular level is not necessary to achieve the desired bonding between the layers.

According to the present invention, the multi-layer topical skin article comprises a skin-contacting layer (bottom) and a outwardly-facing layer (top) opposing the skin-contacting layer and facing away from the skin contacting layer. The skin-contacting layer preferably comprises a hydrophilic film-forming polymer, a solubilizing agent to solubilize other ingredients in the film, a disintegration promoter, a thickening agent/ structuring agent/ texture modifier, a hydroscopic agent/wetting agent to retain skin moisture, a partition coefficient modifier/ absorption-or permeation-promoting substances to drive the hydroscopic agent into skin, a plasticizer/primary adhesive agent for flexibility and softness, a solvent used for hydrocolloids and retain latent moisture and keep final article flexible and other auxiliaries or additives. The skin-contacting layer is applied preferably directly to the skin surface and possesses properties suitable for use as the skin-contacting surface of the article. Such properties include rapid dissolution, sustained adhesion strength, semi-occlusiveness, and flexibility. The skin-contacting layer comprises cosmetically active agents as defined in the claims which can be delivered to the skin by the skin contacting layer.

The outwardly-facing layer possesses proprieties suitable for use as a physical barrier for the multi-layer topical skin article, allowing it to remain clean of dust and dirt and debris while the article remains in place on the application site. Such proprieties include rapid dissolution, semi-occlusiveness, flexibility, and non-stickiness. The outwardly-facing layer comprises a hydrophilic film forming polymer, a disintegration promoter, an oil-in-water emulsifier, a wax to limit water migration from the skin contacting layer to the topical layer, a plasticizer for flexibility and softness, a primary adhesive agent, a solvent used for hydrocolloids and to retain latent moisture and to keep the final article flexible, and other auxiliaries or additives.

The hydrophilic film forming polymers used to prepare the multi-layer topical skin article of the present invention rapidly dissolve in water and form a tacky or sticky gel that readily adheres to the skin surface. The water-dissolvable hydrophilic film forming polymers of the skin-contacting layer interact with the water component in the skin surface moisture through a hydration process upon contact with the skin surface. The hydration force provides an adhesive interaction that aids the skin-contacting layer in adhering to the skin surface.

The hydrophilic film-forming polymers suitable for producing the multi-layer topical skin article may be of synthetic, semisynthetic, or natural origin. Such hydrophilic film forming polymers include, without limitation, cellulose ethers, polyvinyl alcohols, polyvinyl acetate, polyvinyl pyrrolidone, polysaccharides, as well as derivatives, copolymers or polymers thereof. The multi-layer topical skin article may be made into a wide variety of product forms that include but are not limited to the form films.

Adhesion of the film is achieved through a combination of a film former (such as polyvinylpyrrolidone), a tackifier for initial tack or stickiness (such as corn syrup), a plasticizer (such as glycerin) which makes the formula more flexible and helps with solubility and finally, a more hydrophobic ingredient to ensure that the film will substantially maintain its physical properties over a wider range of moisture content.

In one embodiment, adhesion of an article of the present invention is achieved by provision of high fructose corn syrup as an ingredient of the article. The corn syrup, combined with residual moisture remaining in the film after drying, causes the article to exhibit the desired adhesive properties and imparts the adhesive quality to article 100.

Exemplary methods of forming two-layer articles of the present invention are discussed in United States Patent Applications Serial Nos. 14/580,977, entitled "PROCESS FOR FORMING A MULTILAYERED SHAPED FILM PRODUCT", filed on December 23, 2014, and 14/581,010, entitled "SINGLE-PASS PROCESS FOR FORMING A MULTILAYERED SHAPED FILM PRODUCT", filed on December 23, 2014.

An article may be formed in accordance with one embodiment of the present invention by casting and drying the adhesive layer, and then casting the top layer on top of the bottom layer. The two layers adhere to one another by any of the known methods of adhesion (mechanical, chemical, dispersive, electrostatic, diffusive, etc.). In one embodiment, the two layers preferably are both water soluble, so that the water in the nonadhesive outwardly-facing layer will slightly dissolve the already dried adhesive skin contacting layer, thereby creating a certain amount of diffusive adhesion at the interface of the two layers. In a second embodiment of the present invention, both layers are cast wet on wet, and intermixing of the materials occurs at the interface therebetween, thereby creating a bond by diffusive adhesion. Preferably, the materials have a common solvent and / or are miscible with each other so that they intermix and bond together. It will be appreciated that the materials of the adhesive and non-adhesive layers (the skin-contacting and outwardly-facing layers, respectively) could have a common solvent other than water, such as alcohol, so that the materials bond to each other.

Benefiting agents that may be used in articles of the invention include cosmetic agents and therapeutic agents wherein at least one benefiting agent comprises hexylresorcinol in the articles of the invention. As used herein, benefiting agent means an ingredient or material that provides a benefit, e.g., improves, relieves, reduces, or treats symptoms or conditions of the skin, ether cosmetic or therapeutic. Such substances may be any of a variety of compositions, including, without limitation, hyaluronic acid; hydroxyl acids (e.g., glycolic acid, lactic acid, malic acid, salicylic acid, citric acid, tartaric acid); anti-acne agents (e.g., salicylic acid, retinol, retinoids, or other keratolytics, and benzoyl peroxide, or other antimicrobial agents used to treat acne); shine control agents (e.g., rice protein, cotton powder, elubiol (dichlorophenyl-imidazoltioxolan); a retinoid or its derivative such as tretinoin, isotretinoin, motretinide, adapalene, tazarotene, azelaic acid, and retinol; a 5-alpha-reductase inhibitor of amino acids, e.g., glycine derivatives; hydrolyzed vegetable proteins, including soy protein and wheat protein, etc,; green tea *(camellia sinesis)* extract, and cinnamon bark extract); moisturizers; anti-microbial agents (e.g., cationic antimicrobials such as benzylkonium chloride, benzethonium chloride, triclocarbon, polyhexamethylene biguanide, cetylpyridium chloride, methyl and benzothonium chloride; salts of chlorhexidine, such as lodopropynyl butylcarbamate, diazolidinyl urea, chlorhexidene digluconate, chlorhexidene acetate, chlorhexidine isethionate, and chlorhexidene hydrochloride; halogenated phenolic compounds, such as 2,4,4'-trichloro-2-hydroxy diphenyl ether (Triclosan); parachlorometa xylenol (PCMX); short chain alcohols, such as ethanol, propanol, and the like); antibiotics or antiseptics (mupirocin, neomycin sulfate bacitracin, polymyxin B, 1-ofloxacin, tetracyclines (chlortetracycline hydrochloride, oxytetracycline-lOhydrochloride and tetrachcycline hydrochoride), clindamycin phosphate, gentamicin sulfate, metronidazole, hexylresorcinol, methylbenzethonium chloride, phenol, quaternary ammonium compounds, tea tree oil, and their pharmaceutically acceptable salts and prodrugs), anti-inflammatory agents (e.g., suitable steroidal anti-inflammatory agents such as corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinol one acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and salts, nonsteroidal anti-inflammatory agents, feverfew (Tanacetum parthenium), goji berry (Lycium barbarum), milk thistle extract (Silybum marianum), amaranth oil (Amaranthus cruentus), pomegranate (Punica granatum), yerbe mate (Ilex paraguariensis leaf extract), white lily flower extract (Lilium Candidum), olive leaf extract (Olea europaea) and phloretin (apple extract)); anti-mycotic / antifungal agents (e.g., miconazole, econazole, ketoconazole, sertaconazole, itraconazole, fluconazole, voriconazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, and their pharmaceutically acceptable salts and prodrugs; an azole, an allylamine, or a mixture thereof); external analgesics (e.g., ibuprofen- or diclofenac; capsaicin, fentanyl, and salts thereof such fentanyl citrate; paracetamol (as acetaminophen); non-steroidal anti-inflammatory drugs (NSAIDs) such as salicylates; opioid drugs such as morphine and oxycodone; ibuprofen- or diclofenac-containing gel); anti-oxidants (e.g., sulfhydryl compounds and their derivatives (e.g., sodium metabisulfite and N-acetyl cysteine), lipoic acid and dihydrolipoic acid, resveratrol, lactoferrin; ascorbic acid, ascorbic acid esters, and ascorbic acid derivatives (e.g., ascorbyl palmitate and ascorbyl polypeptide); butylhydroxy anisole, butylated hydroxytoluene (butylhydroxy toluene), retinoids (e.g., retinol and retinyl palmitate), tocopherols (e.g., tocopherol acetate), tocotrienols, and ubiquinone; cysteine, N-acetylcysteine, sodium bisulfite, sodium metabisulfite, sodium formaldehydesulfoxylate, acetone sodium bisulfite, tocopherols, and nordihydroguaiaretic acid; extracts containing flavonoids and isoflavonoids and their derivatives (e.g., genistein and diadzein); extracts containing resveratrol and the like; grape seed, green tea, pine bark, and propolis; plant-derived polyphenol antioxidants such as clove, cinnamon, oregano, turmeric, cumin, parsley, basil, curry powder, mustard seed, ginger, pepper, chili powder, paprika, garlic, coriander, onion and cardamom; typical herbs such as sage, thyme, marjoram, tarragon, peppermint, oregano, savory, basil and dill weed)); depilatory agents (e.g., calcium thioglycolate or potassium thioglycolate); vitamins (e.g., Vitamin A, Vitamin B, Vitamins C, Vitamin E; either alpha, beta, gamma or delta tocopherols, niacin or niacinamide) and vitamin salts or derivatives such as ascorbic acid diglucoside and vitamin E acetate or palmitate; sunblock (e.g., titanium dioxide) and / or sunscreen (e.g., inorganic sunscreens such as titanium dioxide and zinc oxide; organic sunscreens such as octyl-methoxy cinnamates, octyl salicylate, homosalate, avobenzone); vasodilators (e.g., niacin); humectants (e.g., glycerin); anti-aging agents (e.g., retinoids; dimethylaminoathanol (DMAE), copper containing peptides); alpha hydroxy acids or fruit acids and their precursors such as glycolic acid, citric acid, lactic acid, malic acid, mandelic acid, ascorbic acid, alpha-hydroxybutyric acid, alpha-hydroxyisobutyric acid, alphahydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, and beta-phenylpyruvic acid; zinc and zinc containing compounds such as zinc oxides; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, and safflower, and salts and prodrugs thereof); carotenoids, ceramides, fatty acids, enzymes, enzyme inhibitors, minerals, steroids, peptides, amino acids, botanical extracts, colorants, etc. The substances may affect the skin in any of a variety of manners, such as by moisturizing; enhancing skin tone or color (such as with pigments); treating or at least mitigating various skin conditions (such as dry or severe dry skin, eczema, psoriasis, atopic dermatitis, allergic rashes, acne, blackheads, pustules, comedones, rosacea, shingles, wrinkles, cold sores, herpes, corns, warts, sunburn, insect bites, poison ivy, etc.); applying a mechanical force to smooth wrinkles; or, more generally, treating or mitigating the symptoms and appearance of undesired skin imperfections (such as under eye dark circle, redness of acne, fine lines and wrinkles, post inflammatory hyperpigmentation (PIH), redness, inflammation, cellulite, wrinkles, age spots, mottled pigmentation, dark spots, liver spots, under eye puffiness); removing unwanted facial or body hair; aiding in wound healing; etc.. For instance, lotions, creams, oils, and even masks may be applied to skin to treat or otherwise to affect the skin. Such personal or consumer healthcare substances are absorbed into the skin generally following the principles of diffusion, under which the rate of diffusion or transport across the skin is correlated with the difference in active concentration on both sides of the skin.

The transfer of the benefiting agent from the article to the mammalian body is enhanced by the use of one or more glyceride emulsifiers in the second bottom layer of the article, such as glycerine derivatives including without limitation, glycerides such as a monoglyceride (e.g., MYVEROL 18-99) and glycerol fatty acid esters such as glycerol oleate (MONOMULS 90-O 18, available from BASF Corporation, Florham Park, New Jersey, USA). It is believed that these components help to release the benefiting agents from the skin-contacting layer into the skin. A user may apply the article to an application site on a mammalian body, such as human skin or mucosal membrane, and allow the article to remain in place for an extended period of time, e.g., at least half an hour, or at least one hour, or at least about six (6) to eight (8) hours, or at least about twelve (12) hours, or about twenty four (24) hours, if desired. Thereafter, the topically-applied article is readily removable upon application of water thereto. By readily removable, it is meant that the article may dissolve or disintegrate upon application of water to the article, such that it may be removed from the skin without scrubbing, or the like. Surprisingly, this extended wear can significantly enhance the efficacy of the skin treatment as shown in the Examples, below.

The present invention will be further understood by reference to the following specific Examples which are illustrative of the composition, form and method of producing the present invention. It is to be understood that many variations of composition, form and method of producing this would be apparent to those skilled in the art. The following Examples, wherein parts and percentages are by weight unless otherwise indicated, are only illustrative.

### Examples

In the following section, reference examples are denoted by an asterisk *

**Table 1: Summary of Films Formed in Examples.**

| **Example** | **Film Function** | **Top Layer Formulation** | **Bottom Layer Formulation** | **Top Layer Film Former** | **Bottom Layer Film Former** | **Active Ingredient** |
|---|---|---|---|---|---|---|
| 1* | Skin Care Base, Moisturizing Film | 12741-093 | 12741-169 | Selvol 805 PVOH | Plasdone K29/32 PVP | Glycerin 99% USP |
| 2 | Hexylresorcinol Anti-aging, Skin Whitening Film | 12741-093 | 12924-021 | Selvol 805 PVOH | Plasdone K29/32 PVP | Hexylresorc inol (0.5%) |
| 3* | Anti-acne Film | 12741-093 | 12924-034 | Selvol 805 PVOH | Plasdone K29/32 PVP | Salicylic Acid |
| 4 | Hexylresorcinol Anti-aging, Skin Whitening Film | 12741-093 | 12924-048 | Selvol 805 PVOH | Plasdone K29/32 PVP | Hexylresorc inol (0.25%) |
| 5* | Anti-wrinkle, Anti-aging Film | 12741-093 | 12924-070 | Selvol 805 PVOH | Plasdone K29/32 PVP | Niacinamid e USP |
| 6* | Ibuprofen Deliverable Film | 12924-135 | 13237-010 | Selvol 805 PVOH | Plasdone K29/32 PVP | Ibupofen USP |
| 7* | ORC Hemostatic Film | 12924-151 | 12924-159 | Selvol 805 PVOH | Plasdone K29/32 PVP | Oxidized Regenerate d Cellulose, Fines 2X |
| 8* | Skin Care Base Film w/ ECD | 12924-120 | 13237-007 | Selvol 805 PVOH | Plasdone K29/32 PVP | None |
| 9 | Hexylresorcinol Anti-aging, Skin Whitening Film w/ECD | 12924-120 | 13237-016 | Selvol 805 PVOH | Plasdone K29/32 PVP | Hexylresorc inol (0.50% ) |
| 10* | Anti-microbial Film | 12924-120 | 13237-021 | Selvol 805 PVOH | Plasdone K29/32 PVP | Polymyxin B Sulfate, Neomycin |
| 11* | Psoriasis Treatment Film | 12924-120 | 13237-023 | Selvol 805 PVOH | Plasdone K29/32 PVP | Refined Coal Tar, 5% |
| 12* | Eczema Treatment Film | 12924-120 | 13237-024 | Selvol 805 PVOH | Plasdone K29/32 PVP | Colloidal Oat Flour |
| 13* | Skin Care Base, Moisturizing Film | 12741-093 | 13237-101 | Selvol 805 PVOH | Plasdone K29/32 PVP and LUVISKOL Plus | Glycerin 99% USP |
| 14 | Hexylresorcinol Anti-aging, Skin Whitening Film | 12741-093 | 13237-129 | Selvol 805 PVOH | Plasdone K29/32 PVP and Plastdone S-630 | Hexylresore inol (0.5%) |

### Example 1*: Moisturizing Film.

**Table 1a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 1b: Formulation for Bottom Layer.**

| ***Notebook #12741, Page #169** | | | | |
|---|---|---|---|---|
| **Ingredients** | **Weight (g)** | **Solid % (in film)** | **% In Solution** | **Lab Batch (g)** |
| Plasdone K-29/32 | 20.6737 | 43.0116 | 20.6737 | 62.0211 |
| Glycerox 767 | 0.9279 | 1.9305 | 0.9279 | 2.7837 |
| Cosmedia SP | 0.6959 | 1.4479 | 0.6959 | 2.0878 |
| Glycerin, USP 99.7% | 4.8065 | 10.0000 | 4.8065 | 14.4196 |
| Myverol 18-99 Monoglycerides | 2.4033 | 5.0000 | 2.4033 | 7.2098 |
| Invertose HFCS 26550 (from Ingredion) | 18.5580 | 38.6100 | 18.5580 | 55.6741 |
| Water, Purified | 51.9347 | | 51.9347 | 155.8040 |
| | | **100.0000** | **100.0000** | |
| **Total Solid Weight (g)** | **48.0654** | | | 144.1961 |
| **Total Batch Weight (g)** | **100.0000** | | | 300.0001 |
| **% Solid** | **48.07** | | | |

### Example 2: Anti-Aging, Skin Whitening Film.

**Table 2a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 2b: Formulation for Bottom Layer.**

| **Notebook #12924, Page #021** | | | | |
|---|---|---|---|---|
| **Ingredients** | **Weight (g)** | **Solid % (in film)** | **% In Solution** | **Lab Batch (g)** |
| Plasdone K-29/32 | 22.2800 | 54.1810 | 25.2833 | 111.4000 |
| Glycerox 767 | 1.0000 | 2.4318 | 1.1348 | 5.0000 |
| Cosmedia SP | 0.7500 | 1.8239 | 0.8511 | 3.7500 |
| Glycerin, USP 99.7% | 7.3297 | 17.8245 | 8.3177 | 36.6485 |
| Myverol 18-99 Monoglycerides | 2.0561 | 5.0001 | 2.3333 | 10.2805 |
| Hexylresorcinol | 0.2056 | 0.5000 | 0.2333 | 1.0280 |
| Invertose HFCS 26550 (Ingredion) | 7.5000 | 18.2387 | 8.5110 | 37.5000 |
| Water, Purified | 47.0000 | | 53.3355 | 235.0000 |
| | | **100.0000** | **100.0000** | |
| **Total Solid Weight (g)** | **41.1214** | | | 205.6070 |
| **Total Batch Weight (g)** | **88.1214** | | | 440.6070 |
| **% Solid** | **46.66** | | | |

### Example 3*: Anti-Acne Film.

**Table 3a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 3b: Formulation for Bottom Layer.**

| **Notebook #12924, Page #034** | | | | |
|---|---|---|---|---|
| **Ingredients (Trade Name)** | **Weight (g)** | **Solid % (in film)** | **% In Solution** | **Lab Batch (g)** |
| Plasdone K-29/32 | 5.0000 | 7.4964 | 4.4763 | 15.0000 |
| Polyox WSR N-10 | 10.2800 | 15.4125 | 9.2033 | 30.8400 |
| Polysorbate 80 | 1.5000 | 2.2489 | 1.3429 | 4.5000 |
| Invertose HFCS 26550 (Ingredion) | 30.0000 | 44.9782 | 26.8579 | 90.0000 |
| Myverol 18-99 | 3.3350 | 5.0001 | 2.9857 | 10.0050 |
| Propylene Glycol | 15.2500 | 22.8639 | 13.6528 | 45.7500 |
| Salicylic Acid, Sal B (from Wego Chem) | 1.3340 | 2.0000 | 1.1943 | 4.0020 |
| Water, Purified | 45.0000 | | 40.2868 | 135.0000 |
| | | **100.0000** | **100.0000** | |
| **Total Solid Weight (g)** | **66.6990** | | | 200.0970 |
| **Total Batch Weight (g)** | **111.6990** | | | 335.0970 |
| **% Solid** | **59.71** | | | |

### Example 4: Anti-Aging, Skin Whitening Film.

**Table 4a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 4b: Formulation for Bottom Layer.**

| **Notebook #12924, Page #048** | | | | |
|---|---|---|---|---|
| **Ingredients** | **Weight (g)** | **Solid % (in film)** | **% In Solution** | **Lab Batch (g)** |
| Plasdone K-29/32 | 22.2800 | 54.3242 | 25.3144 | 111.4000 |
| Glycerox 767 | 1.0000 | 2.4383 | 1.1362 | 5.0000 |
| Cosmedia SP | 0.7500 | 1.8287 | 0.8521 | 3.7500 |
| Glycerin, USP 99.7% | 7.3297 | 17.8717 | 8.3280 | 36.6485 |
| Myverol 18-99 Monoglycerides | 2.0507 | 5.0001 | 2.3300 | 10.2535 |
| Hexylresorcinol | 0.1026 | 0.2502 | 0.1166 | 0.5130 |
| Invertose HFCS 26550 (Ingredion) | 7.5000 | 18.2869 | 8.5215 | 37.5000 |
| Water, Purified | 47.0000 | | 53.4012 | 235.0000 |
| | | **100.0000** | **100.0000** | |
| **Total Solid Weight (g)** | **41.0130** | | | 205.0650 |
| **Total Batch Weight (g)** | **88.0130** | | | 440.0650 |
| **% Solid** | **46.60** | | | |

### Example 5*: Anti-Wrinkle, Anti-Aging Film.

**Table 5a: Formulation for Top Layer.**

| **Notebook # 12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 5b: Formulation for Bottom Layer.**

| **Notebook #12924, Page #070** | | | | |
|---|---|---|---|---|
| **Ingredients** | **Weight (g)** | **Solid % (in film)** | **% In Solution** | **Lab Batch (g)** |
| Plasdone K-29/32 | 20.6737 | 41.4935 | 20.3164 | 62.0210 |
| Glycerox 767 | 0.9279 | 1.8624 | 0.9119 | 2.7837 |
| Cosmedia SP | 0.6959 | 1.3968 | 0.6839 | 2.0878 |
| Glycerin, USP 99.7% | 4.9824 | 10.0000 | 4.8963 | 14.9472 |
| Niacinamide Powder (USP-FCC/Ph.Eur.) from DSM | 1.4947 | 3.0000 | 1.4689 | 4.4841 |
| Myverol 18-99 Monoglycerides | 2.4912 | 5.0000 | 2.4481 | 7.4736 |
| Invertose HFCS 26550 (from Ingredion) | 18.5580 | 37.2473 | 18.2373 | 55.6741 |
| Water, Purified | 51.9347 | | 51.0372 | 155.8040 |
| | | **100.0000** | **100.0000** | |
| **Total Solid Weight (g)** | **49.8238** | | | 149.4715 |
| **Total Batch Weight (g)** | **101.7585** | | | 305.2755 |
| **% Solid** | **48.96** | | | |

### Example 6*: Ibuprofen Delivery Film.

**Table 6a: Formulation for Top Layer.**

| **Notebook #12924, Page #135** | | | | |
|---|---|---|---|---|
| **Ingredients** | **Weight (g)** | **Solid % (in film)** | **% In Solution** | **Lab Batch (g)** |
| Selvol 805 | 22.9730 | 63.1083 | 22.0958 | 68.9189 |
| Polysorbate 80 | 1.3514 | 3.7123 | 1.2998 | 4.0541 |
| Dow Corning 2501 Cosmetic Wax | 0.6757 | 1.8561 | 0.6499 | 2.0270 |
| Kester Wax K-24 | 0.6757 | 1.8561 | 0.6499 | 2.0270 |
| Myverol 18-99 Monoglycerides | 3.9700 | 10.9059 | 3.8184 | 11.9100 |
| Glycerin 99.7%, USP | 6.7568 | 18.5613 | 6.4988 | 20.2703 |
| Water, Purified | 67.5676 | | 64.9876 | 202.7027 |
| | | **100.0000** | **100.0000** | |
| **Total Solid Weight (g)** | **36.4024** | | | 109.2073 |
| **Total Batch Weight (g)** | **103.9700** | | | 311.9100 |
| **% Solid** | **35.01** | | | |

**Table 6b: Formulation for Bottom Layer.**

| **Notebook #13237, Page #010** | | | | |
|---|---|---|---|---|
| **Ingredients** | **Weight (g)** | **Solid % (in film)** | **% In Solution** | **Lab Batch (g)** |
| Plasdone K-29/32 | 20.0000 | 13.3642 | 9.1053 | **30.0000** |
| Cosmedia SP | 1.5000 | 1.0023 | 0.6829 | **2.2500** |
| Glycerox 767 | 1.5000 | 1.0023 | 0.6829 | **2.2500** |
| Glycerin, USP 99.7% | 10.0000 | 6.6821 | 4.5526 | **15.0000** |
| Invertose HFCS 26550 (from Ingredion) | 15.0000 | 10.0232 | 6.8289 | **22.5000** |
| Aquacoat ECD, Ethylcellulose Dispersion (30%) | 30.0000 | 20.0463 | 13.6579 | 45.0000 |
| Triethylamine (TEA) | 1.6533 | 1.1048 | 0.7527 | 2.4800 |
| Ibuprofen USP (Albemarle) | 70.0000 | 46.7748 | 31.8684 | 105.0000 |
| Water, Purified | 70.0000 | | 31.8684 | 105.0000 |
| | | **100.0000** | **100.0000** | |
| **Total Solid Weight (g)** | **149.6533** | | | **224.4800** |
| **Total Batch Weight (g)** | **219.6533** | | | **329.4800** |
| **% Solid** | **68.13** | | | |

### Example 7*: ORC Hemostatic Film.

**Table 7a: Formulation for Top Layer.**

| **Notebook #12924, Page #151** | | | | |
|---|---|---|---|---|
| **Ingredients** | **Weight (g)** | **Solid % (in film)** | **% In Solution** | **Lab Batch (g)** |
| Selvol 805 | 6.0000 | 38.2166 | 14.7420 | 48.0000 |
| Polysorbate 80 | 0.4000 | 2.5478 | 0.9828 | 3.2000 |
| Lubragel MS (Ashland) | 0.8000 | 5.0955 | 1.9656 | 6.4000 |
| Theraclay Green | 0.2000 | 1.2739 | 0.4914 | 1.6000 |
| Veegum HS | 0.8000 | 5.0955 | 1.9656 | 6.4000 |
| Vitacel Oat Fiber HF600-30 (J.Rettenmaier) | 3.7500 | 23.8854 | 9.2138 | 30.0000 |
| Glycerin 99.7%, USP | 3.7500 | 23.8854 | 9.2138 | 30.0000 |
| Water, Purified | 25.0000 | | 61.4251 | 200.0000 |
| | | **100.0000** | **100.0000** | |
| **Total Solid Weight (g)** | **15.7000** | | | 125.6000 |
| **Total Batch Weight (g)** | **40.7000** | | | 325.6000 |
| **% Solid** | **38.57** | | | |

**Table 7b: Formulation for Bottom Layer.**

| **Notebook #12924, Page #159** | | | | |
|---|---|---|---|---|
| **Ingredients** | **Weight (g)** | **Solid % (in film)** | **% In Solution** | **Lab Batch (g)** |
| Plasdone K-29/32 | 20.6737 | 45.1598 | 21.1574 | 62.0211 |
| Glycerox 767 | 0.9279 | 2.0269 | 0.9496 | 2.7837 |
| Cosmedia SP | 0.6959 | 1.5202 | 0.7122 | 2.0878 |
| Glycerin, USP 99.7% | 8.0000 | 17.4753 | 8.1872 | 24.0001 |
| **Oxidized Regenerated Cellulose ORC, Fines 2X (Ethicon)** | **4.5779** | **10.0000** | 4.6850 | 13.7337 |
| **Vitacel oat Fiber HF600-30 (J .Rettenmaier)** | **8.5003** | **18.5681** | 8.6992 | 25.5010 |
| Myverol 18-99 Monoglycerides | 2.4033 | 5.2497 | 2.4595 | 7.2098 |
| Water, Purified | 51.9348 | | 53.1499 | 155.8044 |
| | | **100.0000** | **100.0000** | |
| **Total Solid Weight (g)** | **45.7791** | | | 137.3372 |
| **Total Batch Weight (g)** | **97.7139** | | | 293.1416 |
| **% Solid** | **46.85** | | | |

### Example 8*: Skin Care Base Film.

**Table 8a: Formulation for Top Layer.**

| **Notebook #12924, Page #120** | | | | |
|---|---|---|---|---|
| **Ingredients** | **Weight (g)** | **Solid % (in film)** | **% In Solution** | **Lab Batch (g)** |
| Selvol 805 | 22.9730 | 80.7543 | 27.2037 | 91.8919 |
| Polysorbate 80 | 1.7500 | 6.1516 | 2.0723 | 7.0000 |
| Dow Corning 2501 Cosmetic Wax | 0.8000 | 2.8122 | 0.9473 | 3.2000 |
| Kester Wax K-24 | 0.8000 | 2.8122 | 0.9473 | 3.2000 |
| Glycerin 99.7%, USP | 2.1250 | 7.4698 | 2.5163 | 8.5000 |
| Water, Purified | 56.0000 | | 66.3130 | 224.0000 |
| | | **100.0000** | **100.0000** | |
| **Total Solid Weight (g)** | **28.4480** | | | 113.7919 |
| **Total Batch Weight (g)** | **84.4480** | | | 337.7919 |
| **% Solid** | **33.69** | | | |

**Table 8b: Formulation for Bottom Layer.**

| **Notebook #13237, Page #007** | | | | |
|---|---|---|---|---|
| **Ingredients** | **Weight (g)** | **Solid % (in film)** | **% In Solution** | **Lab Batch (g)** |
| Plasdone K-29/32 | 20.6737 | 28.1055 | 16.4741 | 62.0210 |
| Glycerox 767 | 0.9279 | 1.2615 | 0.7394 | 2.7837 |
| Cosmedia SP | 0.6959 | 0.9461 | 0.5546 | 2.0878 |
| Glycerin, USP 99.7% | 4.8065 | 6.5344 | 3.8301 | 14.4196 |
| Myverol 18-99 Monoglycerides | 2.4033 | 3.2672 | 1.9151 | 7.2098 |
| Aquacoat ECD, Ethylcellulose Dispersion (30%) | 25.4920 | 34.6560 | 20.3137 | 76.4759 |
| Invertose HFCS 26550 (from Ingredion) | 18.5580 | 25.2293 | 14.7882 | 55.6740 |
| Water, Purified | 51.9346 | | 41.3848 | 155.8037 |
| | | **100.0000** | **100.0000** | |
| **Total Solid Weight (g)** | **73.5572** | | | 220.6717 |
| **Total Batch Weight (g)** | **125.4918** | | | 376.4754 |
| **% Solid** | **58.62** | | | |

### Example 9: Anti-Aging, Skin Whitening Film.

**Table 9a: Formulation for Top Layer.**

| **Notebook #12924, Page #120** | | | | |
|---|---|---|---|---|
| **Ingredients** | **Weight (g)** | **Solid % (in film)** | **% In Solution** | **Lab Batch (g)** |
| Selvol 805 | 22.9730 | 80.7543 | 27.2037 | 91.8919 |
| Polysorbate 80 | 1.7500 | 6.1516 | 2.0723 | 7.0000 |
| Dow Corning 2501 Cosmetic Wax | 0.8000 | 2.8122 | 0.9473 | 3.2000 |
| Kester Wax K-24 | 0.8000 | 2.8122 | 0.9473 | 3.2000 |
| Glycerin 99.7%, USP | 2.1250 | 7.4698 | 2.5163 | 8.5000 |
| Water, Purified | 56.0000 | | 66.3130 | 224.0000 |
| | | **100.0000** | **100.0000** | |
| **Total Solid Weight (g)** | **28.4480** | | | 113.7919 |
| **Total Batch Weight (g)** | **84.4480** | | | 337.7919 |
| **% Solid** | **33.69** | | | |

**Table 9b: Formulation for Bottom Layer.**

| **Notebook #13237, Page #016** | | | | |
|---|---|---|---|---|
| **Ingredients** | **Weight (g)** | **Solid % (in film)** | **% In Solution** | **Lab Batch (g)** |
| Plasdone K-29/32 | 20.6737 | 25.4062 | 15.7367 | 62.0210 |
| Glycerox 767 | 1.0000 | 1.2289 | 0.7612 | 3.0000 |
| Cosmedia SP | 0.8000 | 0.9831 | 0.6090 | 2.4000 |
| Glycerin, USP 99.7% | 15.0000 | 18.4337 | 11.4179 | 45.0000 |
| Myverol 18-99 Monoglycerides | 3.0000 | 3.6867 | 2.2836 | 9.0000 |
| Hexylresorcinol | 0.4069 | 0.5000 | 0.3097 | 1.2207 |
| Aquacoat ECD, Ethylcellulose Dispersion (30%) | 25.4920 | 31.3275 | 19.4043 | 76.4759 |
| Invertose HFCS 26550 (from Ingredion) | 15.0000 | 18.4337 | 11.4179 | 45.0000 |
| Water, Purified | 50.0000 | | 38.0597 | 150.0000 |
| | | **100.0000** | **100.0000** | |
| **Total Solid Weight (g)** | **81.3725** | | | 244.1176 |
| **Total Batch Weight (g)** | **131.3725** | | | 394.1176 |
| **% Solid** | **61.94** | | | |

### Example 10*: Anti-Microbial Film.

**Table 10a: Formulation for Top Layer.**

| **Notebook #12924, Page #120** | | | | |
|---|---|---|---|---|
| **Ingredients** | **Weight (g)** | **Solid % (in film)** | **% In Solution** | **Lab Batch (g)** |
| Selvol 805 | 22.9730 | 80.7543 | 27.2037 | 91.8919 |
| Polysorbate 80 | 1.7500 | 6.1516 | 2.0723 | 7.0000 |
| Dow Corning 2501 Cosmetic Wax | 0.8000 | 2.8122 | 0.9473 | 3.2000 |
| Kester Wax K-24 | 0.8000 | 2.8122 | 0.9473 | 3.2000 |
| Glycerin 99.7%, USP | 2.1250 | 7.4698 | 2.5163 | 8.5000 |
| Water, Purified | 56.0000 | | 66.3130 | 224.0000 |
| | | **100.0000** | **100.0000** | |
| **Total Solid Weight (g)** | **28.4480** | | | 113.7919 |
| **Total Batch Weight (g)** | **84.4480** | | | 337.7919 |
| **% Solid** | **33.69** | | | |

**Table 10b: Formulation for Bottom Layer.**

| **Notebook #13237, Page #021** | | | | |
|---|---|---|---|---|
| **Ingredients** | **Weight (g)** | **Solid % (in film)** | **% In Solution** | **Lab Batch (g)** |
| Plasdone K-29/32 | 18.6737 | 25.2157 | 16.3724 | 56.0210 |
| Glycerox 767 | 0.9279 | 1.2530 | 0.8136 | 2.7837 |
| Cosmedia SP | 0.6959 | 0.9397 | 0.6102 | 2.0878 |
| Glycerin, USP 99.7% | 10.8065 | 14.5925 | 9.4748 | 32.4196 |
| Myverol 18-99 Monoglycerides | 2.4033 | 3.2452 | 2.1071 | 7.2098 |
| Lubragel MS (Ashland) | 4.0000 | 5.4014 | 3.5071 | 12.0000 |
| Polymyxin B Sulfate | 0.0980 | 0.1323 | 0.0859 | 0.2940 |
| Neomycin | 0.4003 | 0.5405 | 0.3510 | 1.2009 |
| Aquacoat ECD, Ethylcellulose Dispersion (30%) | 21.4920 | 29.0214 | 18.8434 | 64.4759 |
| Manuka Honey, 100% Pure (Manuka Health New Zealand) | 14.5580 | 19.6582 | 12.7640 | 43.6740 |
| Water, Purified | 40.0000 | | 35.0706 | 120.0000 |
| | | 100.0000 | 100.0000 | |
| **Total Solid Weight (g)** | 74.0555 | | | 222.1666 |
| **Total Batch Weight (g)** | 114.0555 | | | 342.1666 |
| **% Solid** | 64.93 | | | |

### Example 11*: Psoriasis Treatment Film.

**Table 11a: Formulation for Top Layer.**

| **Notebook #12924, Page #120** | | | | |
|---|---|---|---|---|
| **Ingredients** | **Weight (g)** | **Solid % (in film)** | **% In Solution** | **Lab Batch (g)** |
| Selvol 805 | 22.9730 | 80.7543 | 27.2037 | 91.8919 |
| Polysorbate 80 | 1.7500 | 6.1516 | 2.0723 | 7.0000 |
| Dow Corning 2501 Cosmetic Wax | 0.8000 | 2.8122 | 0.9473 | 3.2000 |
| Kester Wax K-24 | 0.8000 | 2.8122 | 0.9473 | 3.2000 |
| Glycerin 99.7%, USP | 2.1250 | 7.4698 | 2.5163 | 8.5000 |
| Water, Purified | 56.0000 | | 66.3130 | 224.0000 |
| | | **100.0000** | **100.0000** | |
| **Total Solid Weight (g)** | **28.4480** | | | 113.7919 |
| **Total Batch Weight (g)** | **84.4480** | | | 337.7919 |
| **% Solid** | **33.69** | | | |

**Table 11b: Formulation for Bottom Layer.**

| **Notebook #13237 Page #023** | | | | |
|---|---|---|---|---|
| **Ingredients** | **Weight (g)** | **Solid % (in film)** | **% In Solution** | **Lab Batch (g)** |
| Plasdone K-29/32 | 18.6737 | 25.5041 | 16.4935 | 56.0210 |
| Glycerox 767 | 0.9279 | 1.2673 | 0.8196 | 2.7837 |
| Cosmedia SP | 0.6959 | 0.9505 | 0.6147 | 2.0878 |
| Glycerin, USP 99.7% | 10.8065 | 14.7594 | 9.5449 | 32.4196 |
| Myverol 18-99 Monoglycerides | 2.4033 | 3.2823 | 2.1227 | 7.2098 |
| Refined Coal Tar, (The Lubrizol Corporation) | 3.6609 | 5.0000 | 3.2335 | 10.9827 |
| Aquacoat ECD, Ethylcellulose Dispersion (30%) | 21.4920 | 29.3533 | 18.9828 | 64.4759 |
| Invertose HFCS 26550 (from Ingredion) | 14.5580 | 19.8831 | 12.8584 | 43.6740 |
| Water, Purified | 40.0000 | | 35.3300 | 120.0000 |
| | | 100.0000 | 100.0000 | |
| **Total Solid Weight (g)** | 73.2181 | | | 219.6544 |
| **Total Batch Weight (g)** | 113.2181 | | | 339.6544 |
| **% Solid** | 64.67 | | | |

### Example 12*: Eczema Treatment Film.

**Table 12a: Formulation for Top Layer.**

| **Notebook #12924, Page #120** | | | | |
|---|---|---|---|---|
| **Ingredients** | **Weight (g)** | **Solid % (in film)** | **% In Solution** | **Lab Batch (g)** |
| Selvol 805 | 22.9730 | 80.7543 | 27.2037 | 91.8919 |
| Polysorbate 80 | 1.7500 | 6.1516 | 2.0723 | 7.0000 |
| Dow Corning 2501 Cosmetic Wax | 0.8000 | 2.8122 | 0.9473 | 3.2000 |
| Kester Wax K-24 | 0.8000 | 2.8122 | 0.9473 | 3.2000 |
| Glycerin 99.7%, USP | 2.1250 | 7.4698 | 2.5163 | 8.5000 |
| Water, Purified | 56.0000 | | 66.3130 | 224.0000 |
| | | **100.0000** | **100.0000** | |
| **Total Solid Weight (g)** | **28.4480** | | | 113.7919 |
| **Total Batch Weight (g)** | **84.4480** | | | 337.7919 |
| **% Solid** | **33.69** | | | |

**Table 12b: Formulation for Bottom Layer.**

| **Notebook #13237 Page #024** | | | | |
|---|---|---|---|---|
| **Ingredients** | **Weight (g)** | **Solid % (in film)** | **% In Solution** | **Lab Batch (g)** |
| Plasdone K-29/32 | 18.6737 | 25.5041 | 16.4935 | 56.0210 |
| Glycerox 767 | 0.9279 | 1.2673 | 0.8196 | 2.7837 |
| Cosmedia SP | 0.6959 | 0.9505 | 0.6147 | 2.0878 |
| Glycerin, USP 99.7% | 10.8065 | 14.7594 | 9.5449 | 32.4196 |
| Myverol 18-99 Monoglycerides | 2.4033 | 3.2823 | 2.1227 | 7.2098 |
| Colloidal Oat Flour | 3.6609 | 5.0000 | 3.2335 | 10.9827 |
| Aquacoat ECD, Ethylcellulose Dispersion (30%) | 21.4920 | 29.3533 | 18.9828 | 64.4759 |
| Invertose HFCS 26550 (from Ingredion) | 14.5580 | 19.8831 | 12.8584 | 43.6740 |
| Water, Purified | 40.0000 | | 35.3300 | 120.0000 |
| | | 100.0000 | 100.0000 | |
| **Total Solid Weight (g)** | 73.2181 | | | 219.6544 |
| **Total Batch Weight (g)** | 113.2181 | | | 339.6544 |
| **% Solid** | 64.67 | | | |

### Example 13*: Moisturizing Film.

**Table 13a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 13b: Formulation for Bottom Layer.**

| **Notebook #13237, Page #101** | | | | |
|---|---|---|---|---|
| **Ingredients** | **Weight (g)** | **Solid % (in film)** | **% In Solution** | **Lab Batch (g)** |
| Plasdone K-29/32 | 17.824 | 43.5684 | 20.2752 | 60.8256 |
| Luviskol Plus (polyvinylcaprolactam, BASF) | 4.4560 | 10.8921 | 5.0688 | 15.2064 |
| Glycerox 767 | 1.0000 | 2.4444 | 1.1375 | 3.4126 |
| Cosmedia SP | 0.7500 | 1.8333 | 0.8531 | 2.5594 |
| Glycerin, USP 99.7% | 7.3297 | 17.9165 | 8.3377 | 25.0131 |
| Myverol 18-99 Monoglycerides | 2.0507 | 5.0127 | 2.3327 | 6.9981 |
| Invertose HFCS 26550 (from Ingredion) | 7.5000 | 18.3327 | 8.5314 | 25.5942 |
| Water, Purified | 47.0000 | | 53.4635 | 160.3906 |
| | | **100.0000** | **100.0000** | |
| **Total Solid Weight (g)** | **40.9104** | | | 139.6094 |
| **Total Batch Weight (g)** | **87.9104** | | | 300.0000 |
| **% Solid** | **46.54** | | | |

### Example 14: Anti-Aging, Skin Whitening Film.

**Table 14a: Formulation for Top Layer.**

| **Notebook #12741, Page #093** | | | | |
|---|---|---|---|---|
| **Ingredients** | **Weight (g)** | **Solid % (in film)** | **% In Solution** | **Lab Batch (g)** |
| Selvol 805 | 22.9730 | 70.8333 | 22.9730 | 68.9189 |
| Polysorbate 80 | 1.3514 | 4.1667 | 1.3514 | 4.0541 |
| Dow Corning 2501 Cosmetic Wax | 0.6757 | 2.0833 | 0.6757 | 2.0270 |
| Kester Wax K-24 | 0.6757 | 2.0833 | 0.6757 | 2.0270 |
| Glycerin 99.7%, USP | 6.7568 | 20.8333 | 6.7568 | 20.2703 |
| Water, Purified | 67.5676 | | 67.5676 | 202.7027 |
| | | **100.0000** | **100.0000** | |
| **Total Solid Weight (g)** | **32.4324** | | | 97.2973 |
| **Total Batch Weight (g)** | **100.0000** | | | 300.0000 |
| **% Solid** | **32.43** | | | |

**Table 14b: Formulation for Bottom Layer.**

| **Notebook #13237, Page #129** | | | | |
|---|---|---|---|---|
| **Ingredients** | **Weight (g)** | **Solid % (in film)** | **% In Solution** | **Lab Batch (g)** |
| Plasdone K-29/32 | 17.8240 | 43.3448 | 20.2266 | 70.7932 |
| Plasdone S-630 | 4.4560 | 10.8362 | 5.0567 | 17.6983 |
| Glycerox 767 | 1.0000 | 2.4318 | 1.1348 | 3.9718 |
| Cosmedia SP | 0.7500 | 1.8239 | 0.8511 | 2.9788 |
| Glycerin, USP 99.7% | 7.3297 | 17.8245 | 8.3177 | 29.1121 |
| Monomuls 90-018 | 2.0561 | 5.0001 | 2.3333 | 8.1664 |
| Hexylresorcinol | 0.2056 | 0.5000 | 0.2333 | 0.8166 |
| Invertose HFCS 26550 (Ingredion) | 7.5000 | 18.2387 | 8.5110 | 29.7558 |
| Water, Purified | 47.0000 | | 53.3355 | 186.7643 |
| | | **100.0000** | **100.0000** | |
| **Total Solid Weight (g)** | **41.1214** | | | 163.3257 |
| **Total Batch Weight (g)** | **88.1214** | | | 350.0000 |
| **% Solid** | **46.66** | | | |

### Example 15

A two-cell clinical study compared a placebo article and an article containing hexylresorcinol over the course of eight weeks. There were 15 subjects per cell who were given a film to place on the dark spot on their face every other night and washed off in the morning. Moisture benefits of the film were evaluated using the MoistureMeterD XS5 probe (Delfin Technologies, Kuopio, Finland) and perceived benefits were measured through individual subject questionnaires.

The both test cell articles were formed of two layers and the top layer for each test cell was the same:
Formulation for Top Layer.

**Table 15a: Formulation for Top Layer.**

| **Formulation** 12924-120 | |
|---|---|
| **Ingredients** | **Formula Weight %** |
| Selvol 805 | 27.20 |
| Polysorbate 80 | 2.07 |
| Dow Corning 2501 Cosmetic Wax | 0.95 |
| Kester Wax K-24 | 0.95 |
| Glycerin 99.7%, USP | 2.52 |
| Water, Purified | 66.31 |
| | **100** |

Bottom Formulation for Placebo Article

**Table 15b: Formulation for Bottom Layer.**

| **Formulation** 13237-027 | |
|---|---|
| **Ingredients** | **Formula Weight %** |
| Plasdone K-29/32 | 25.34 |
| Glycerox 767 | 1.14 |
| Cosmedia SP | 0.85 |
| Glycerin, USP 99.7% | 8.34 |
| Myverol 18-99 Monoglycerides | 2.33 |
| Invertose HFCS 26550 (from Ingredion) | 8.53 |
| Water, Purified | 53.46 |
| | **100** |

Bottom Formulation for Inventive Article

**Table 15c: Formulation for Bottom Layer.**

| **Formulation** 12924-021 | |
|---|---|
| **Ingredients** | **Formula Weight %** |
| Plasdone K-29/32 | 25.28 |
| Glycerox 767 | 1.13 |
| Cosmedia SP | 0.85 |
| Glycerin, USP 99.7% | 8.32 |
| Myverol 18-99 Monoglycerides | 2.33 |
| Synovea HR Hexylresorcinol | 0.23 |
| Invertose HFCS 26550 (from Ingredion) | 8.51 |
| Water, Purified | 53.34 |
| | **100** |

After 8 weeks of use, 81.3% of subjects who used the Hexylresorcinol spot treatment agreed that the tone-correcting product helped visibly reduce the appearance of their dark spot as compared to 23.1% of subjects who used the Placebo spot treatment.

After 8 weeks, 100% of subjects demonstrated an increase in the moisture content of their skin.

### Example 16

A comparison was made between articles incorporating glycerin derivative emulsifiers and articles without such emulsifiers with a control commercially available Hexylresorcinol product (NEUTROGENA® Triple Age Repair). The top layer for all articles was the same as Example 15 (Formulation 1294-120, Table 15a).

The formulations for the bottom layers are as follows:
Example 16A (Hexylresorcinol/Myverol 18-99), Formulation 12924-021

**Table 16a: Formulation for Bottom Layers of Example 16A (Hexylresorcinol/Myverol 18-99).**

| **Notebook #12924, Page #021** | | | | |
|---|---|---|---|---|
| **Ingredients** | **Weight (g)** | **Solid % (in film)** | **% In Solution** | **Lab Batch (g)** |
| Plasdone K-29/32 | 22.2800 | 54.1810 | 25.2833 | 111.4000 |
| Glycerox 767 | 1.0000 | 2.4318 | 1.1348 | 5.0000 |
| Cosmedia SP | 0.7500 | 1.8239 | 0.8511 | 3.7500 |
| Glycerin, USP 99.7% | 7.3297 | 17.8245 | 8.3177 | 36.6485 |
| Myverol 18-99 Monoglycerides | 2.0561 | 5.0001 | 2.3333 | 10.2805 |
| Hexylresorcinol | 0.2056 | 0.5000 | 0.2333 | 1.0280 |
| Invertose HFCS 26550 (Ingredion) | 7.5000 | 18.2387 | 8.5110 | 37.5000 |
| Water, Purified | 47.0000 | | 53.3355 | 235.0000 |
| | | **100.0000** | **100.0000** | |
| **Total Solid Weight (g)** | **41.1214** | | | 205.6070 |
| **Total Batch Weight (g)** | **88.1214** | | | 440.6070 |
| **% Solid** | **46.66** | | | |

Example 16B (Hexylresorcinol/Monomuls 90-018), Formulation 12924-021

**Table 16b: Formulation for Bottom Layers of Example 16A (Hexylresorcinol/Monomuls 90-018).**

| **Notebook #13237, Page #103** | | | | |
|---|---|---|---|---|
| **Ingredients** | **Weight (g)** | **Solid % (in film)** | **% In Solution** | **Lab Batch (g)** |
| Plasdone K-29/32 | 22.2800 | 54.1810 | 25.2833 | 101.1332 |
| Glycerox 767 | 1.0000 | 2.4318 | 1.1348 | 4.5392 |
| Cosmedia SP | 0.7500 | 1.8239 | 0.8511 | 3.4044 |
| Glycerin, USP 99.7% | 7.3297 | 17.8245 | 8.3177 | 33.2709 |
| Monomuls 90-018 (BASF) | 2.0561 | 5.0001 | 2.3333 | 9.3330 |
| Hexylresorcinol | 0.2056 | **0.5000** | 0.2333 | 0.9333 |
| Invertose HFCS 26550 (Ingredion) | 7.5000 | 18.2387 | 8.5110 | 34.0439 |
| Water, Purified | 47.0000 | | 53.3355 | 213.3420 |
| | | **100.0000** | **100.0000** | |
| **Total Solid Weight (g)** | **41.1214** | | | 186.6580 |
| **Total Batch Weight (g) % Solid** | **88.1214** | | | 400.0000 |
| | **46.66** | | | |
| | | | | |

*Example 16C (Hexylresorcinol/No Emulsifier), Formulation 13237-097

**Table 16c: Formulation for Bottom Layers of Example 16A (Hexylresorcinol/Monomuls 90-018).**

| **Notebook #13237, Page #097** | | | | |
|---|---|---|---|---|
| **Ingredients** | **Weight (g)** | **Solid % (in film)** | **% In Solution** | **Lab Batch (g)** |
| Plasdone K-29/32 | 22.2800 | 57.0478 | 25.8904 | 111.4000 |
| Glycerox 767 | 1.0000 | 2.5605 | 1.1620 | 5.0000 |
| Cosmedia SP | 0.7500 | 1.9204 | 0.8715 | 3.7500 |
| Glycerin, USP 99.7% | 7.3297 | 18.7676 | 8.5175 | 36.6485 |
| Emulsifier | 0.0000 | 0.0000 | 0.0000 | 0.0000 |
| Hexylresorcinol | 0.1953 | **0.5001** | 0.2269 | 0.9765 |
| Invertose HFCS 26550 (Ingredion) | 7.5000 | 19.2037 | 8.7154 | 37.5000 |
| Water, Purified | 47.0000 | | 54.6162 | 235.0000 |
| | | **100.0000** | **100.0000** | |
| **Total Solid Weight (g)** | **39.0550** | | | 195.2750 |
| **Total Batch Weight (g) % Solid** | **86.0550** | | | 430.2750 |
| | **45.38** | | | |

These products were tested by the following protocol:
- Wash Franz cells with ethanol 70%, water and receptor solution (IX PBS, phosphate buffered saline).
- Measure skin thickness and electrical resistance
- Mount the tissue on Franz cells (n=4).
- Once equilibrated, make sure there isn't excess moisture on the surface of the tissue (SC) by dabbing the skin surface with Kimwipes.
- Carefully remove the backing on the film and place it on the donor area with a light pressure (5 times tap on skin surface); the adhesive side is in touch with skin.
- Mount the donor chamber and leave it overnight
- Next morning (After 16 h time point), take a sample (300 uL) (0h), replace with fresh IX PBS.
- Add 100 uL IX PBS to the donor chamber, let it sit for 30 sec, remove it using a plastic pipet moving around the edge of the donor. Repeat this cycle 5 times. Pool these 1X PBS solution and analyze by HPLC
- Remove the donor chamber with the tissue still mounted on the Franz cells.
   Use a cotton swab to take out all the remained liquid on skin. Extract drug from this swab by shaking at 100 rpm overnight in 2 mL ethanol: Water (50:50 v/v).
- Put the donor chamber back. After every 2 hours (2,4,6 h), take sample (300 uL) and replace with fresh receptor solution.
- At 22 h, remove the donor chamber, remove skin from cells, put skin on sheets of paper and do tape stripping with 2 tapes to remove the excessive drug on skin surface. Put these 2 tapes in a well in 6-well plate for extraction.
- Use forceps to separate epidermis and dermis. Mince them by scissors. Pool them separately into 6 well plates.
- Add 2 ml ethanol: Water (50:50 v/v) for extraction overnight, 100 rpm.
- Filter the samples through 0.45 um filter and run HPLC

### Film extraction study

- Separate 3 films from the backing layer (n=3)
- Place them separately in 6 well plate
- Add 2 ml ethanol: Water (50:50 v/v) and do extraction overnight at 100 rpm as for skin extraction.
- Inject in HPLC and analyze the results.

**Note:** Standard curve and extraction solvent is ethanol: water (50:50 v/v)

**On 6 well plate:**
- 2 tapes
- Epidermis
- Dermis
- Cotton swabs

| **EXPERIMENT PARAMETERS** | |
|---|---|
| **HPLC METHOD** | |
| HPLC | Water e2795 Separations Module |
| Detector | Water 2998 Photodiode Arrav Detector |
| Column | Waters Sun Fire™ C18 column (3.5 um, 4.6 x 150 mm) |
| Pump mode | Isocratic |
| Mobile phase | Methanol: DI Water (0.05% TFA) (75:25 v/v) |
| Comlumn Temp | 40 degree Celcius |
| Wavelength | 280 nm |
| Injection volume | 35 uL |
| Flow rate | 1.0 ml/min |
| Run time | 10 min |
| Retention time | 4.9 min |

NEUTROGENA® Triple Age Repair (1% Hexylresorcinol), was used as a topical control, and it was applied to the test tissue in a similar manner to the test film articles.

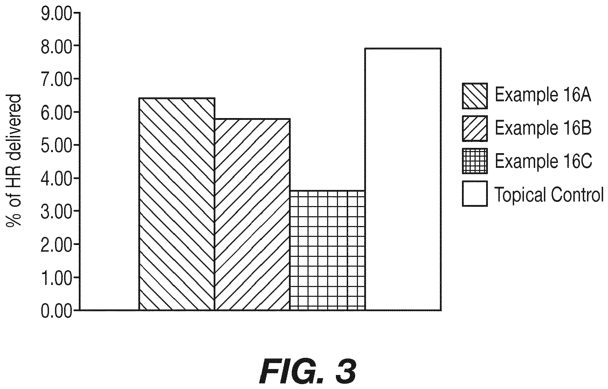

The results are shown graphically in Fig. 3. It can be seen that the use of the emulsifier (Myverol or Monumuls) greatly enhanced the delivery of the hexylresorcinol compared to Example 16C. In addition, the hexylresorcinol delivered via the film articles of Examples 16A and 16B are similar to that of a topical.

## Claims

1. An article suitable for providing a cosmetic and/or therapeutic benefit to the skin upon topical application of said article thereto, comprising:
a first top layer having a top surface facing outwardly from said skin and a second bottom surface, opposite said top surface, facing towards said skin,
a second bottom layer comprising a first surface facing and adhered to said bottom surface of said first top layer and a second outwardly-facing surface for contacting and adhering said article to said skin when said article is applied thereto, said second bottom layer comprising at least one benefiting agent comprising hexylresorcinol,
wherein each of said first top layer and said second bottom layer comprises a water-soluble film former and said article is readily removable from said skin upon application of water thereto,
wherein the second bottom layer further comprises an effective amount of a glyceride emulsifier to enhance transport of the benefiting agent therefrom to the skin.

2. The article of claim 1 wherein the at least one benefiting agent comprises a plurality of benefiting agents.

3. The article of claim 1 wherein the top layer comprises polyvinyl alcohol.

4. The article of claim 1 wherein the bottom layer comprises polyvinyl pyrrolidone.

5. A non-therapeutic method of providing a cosmetic benefit to skin, the method comprising:
applying an article according to claim 1, wherein the at least one benefiting agent comprises hexylresorcinol, to an application site on the skin;
maintaining the article in place for at least half an hour; and
removing the article from the application site by application of water to the article.

## Patentansprüche

1. Artikel, geeignet für die Bereitstellung eines kosmetischen und/oder therapeutischen Nutzens für die Haut bei topischer Anwendung des Artikels darauf, umfassend:
eine erste obere Schicht mit einer von der Haut abgewandten oberen Oberfläche und einer der oberen Oberfläche gegenüberliegenden, zur Haut gerichteten zweiten unteren Oberfläche,
wobei eine zweite untere Schicht eine erste Oberfläche, die der unteren Oberfläche der ersten oberen Schicht gegenüberliegt und daran haftet, und eine zweite, nach außen gerichtete Oberfläche zum Inkontaktbringen des Artikels mit der Haut und zum Anhaften des Artikels an der Haut bei der Anwendung des Artikels darauf umfasst, wobei die zweite untere Schicht mindestens ein Hexylresorcinol umfassendes nutzbringendes Mittel umfasst,
wobei die erste obere Schicht und die zweite untere Schicht jeweils einen wasserlöslichen Filmbildner umfassen und der Artikel bei Anwendung von Wasser darauf leicht von der Haut entfernbar ist,
wobei die zweite untere Schicht weiterhin eine wirksame Menge eines Glycerid-Emulgators zur Verbesserung des Transports des nutzbringenden Mittels davon zur Haut umfasst.

2. Artikel nach Anspruch 1, wobei das mindestens eine nutzbringende Mittel mehrere nutzbringende Mittel umfasst.

3. Artikel nach Anspruch 1, wobei die obere Schicht Polyvinylalkohol umfasst.

4. Artikel nach Anspruch 1, wobei die untere Schicht Polyvinylpyrrolidon umfasst.

5. Nichttherapeutisches Verfahren zur Bereitstellung eines kosmetischen Nutzens für Haut, wobei das Verfahren Folgendes umfasst:
die Anwendung eines Artikels nach Anspruch 1, wobei das mindestens eine nutzbringende Mittel Hexylresorcinol umfasst auf einer Anwendungsstelle auf der Haut,
das an der Stelle Halten des Artikels für mindestens eine halbe Stunde und
das Entfernen des Artikels von der Anwendungsstelle durch Anwendung von Wasser auf den Artikel.

## Revendications

1. Article approprié pour fournir un bénéfice cosmétique et/ou thérapeutique à la peau par une application topique dudit article à celle-ci, comprenant :
une première couche supérieure possédant une surface supérieure tournée vers l'extérieur depuis ladite peau et une deuxième surface inférieure, opposée à ladite surface supérieure, tournée vers ladite peau,
une deuxième couche inférieure comprenant une première surface tournée vers et collant à ladite surface inférieure de ladite première couche supérieure et une deuxième surface tournée vers l'extérieur pour la mise en contact et l'adhérence dudit article à ladite peau lorsque ledit article est appliqué à celle-ci, ladite deuxième couche inférieure comprenant au moins un agent bénéfique comprenant de l'hexylrésorcinol,
chacune parmi ladite première couche supérieure et ladite deuxième couche inférieure comprenant un agent filmogène soluble dans l'eau et ledit article étant aisément détachable de ladite peau lors de l'application d'eau à celle-ci,
la deuxième couche inférieure comprenant en outre une quantité efficace d'un émulsifiant de type glycéride pour augmenter le transport de l'agent bénéfique depuis celle-ci à la peau.

2. Article selon la revendication 1, l'au moins un agent bénéfique comprenant une pluralité d'agents bénéfiques.

3. Article selon la revendication 1, la couche supérieure comprenant un poly(alcool vinylique).

4. Article selon la revendication 1, la couche inférieure comprenant une polyvinylpyrrolidone.

5. Procédé non thérapeutique de fourniture d'un bénéfice cosmétique à la peau, le procédé comprenant :
l'application d'un article selon la revendication 1, l'au moins un agent bénéfique comprenant de l'hexylrésorcinol, à un site d'application sur la peau ,
le maintien en place de l'article pendant au moins une demi-heure ; et
le retrait de l'article du site d'application par l'application d'eau à l'article.
